# EUROPEAN PATENT SPECIFICATION

(11) **EP 1 208 080 B1**
(45) Date of publication and mention of the grant of the patent: **14.01.2004**
(21) Application number: 99939075.0
(22) Date of filing: 09.08.1999
(51) Int. Cl.: C07C 233/47, C11D 1/10, A61K 7/50

(54) **PROCESS FOR MAKING SALT-FREE AMPHOTERICS WITH HIGH MONO AMPHOPROPIONATE CONTENT**
VERFAHREN ZUR HERSTELLUNG VON SALZFREIEN AMPHOTHEREN MIT HOHEM MONO-AMPHOPROPIONATGEHALT
PROCEDE DE PRODUCTION D'AMPHOTERES SANS SELS AYANT UNE TENEUR ELEVEE EN MONOAMPHOPROPIONATE

(43) Date of publication of application: 29.05.2002
(73) Proprietor: RHODIA INC., Cranbury, New Jersey 08512-7500 (US)
(72) Inventor: WO, Shiming, Hightstown, NJ 08520 (US); LI, Ji, F-69110 Ste-Foy-Les-Lyon (FR); HASHEM, Mohamed, Robbinsville, NJ 08691 (US); VUKOV, Rastko, Princeton, NJ 08540 (US)
(74) Representative: Kyle, Diana
(86) International application number: PCT/US1999/017921
(87) International publication number: WO 2001/010820

(56) References cited:
- EP-A- 0 224 796
- EP-A- 0 269 939
- EP-A- 0 595 383
- EP-A- 0 640 334
- DE-A- 3 639 752
- DATABASE WPI Section Ch, Week 199812 Derwent Publications Ltd., London, GB; Class E12, AN 1998-126468 XP002134774 & JP 10 008086 A (ADEKA CLEAN AID KK), 13 January 1998 (1998-01-13)

## Description

The present invention relates generally to surfactants and cleaning compositions useful in cosmetic and personal care applications such as soaps shampoos, toiletries and the like. In particular, the present invention relates to the preparation of these compositions and an improved process that is both user and environmental friendly.

### BACKGROUND OF THE INVENTION

Surfactants, or surface active agents, are useful in cleaning compositions as they reduce the intermolecular attraction of one compound or material from that of another. In other words, they reduce the surface tension that exists between dirt, oil or grease and the skin, hair, or some other inert material such as porcelain, fabric, hard surfaces and the like. In so doing, the dirt or grease is released from the surface of the second material which is consequently cleaned.

There are three basic types of surfactant and many different species of each. Detergents reduce the surface tension of water and specifically exert emulsifying action at oil-water interfaces and in this way function to remove soils. Emulsifiers are basically a type of detergent and hold two or more liquids in suspension. Wetting agents reduce the surface tension of water whereby it is able to more easily penetrate or spread over the surface of another material.

Surfactants can also be classified in terms of their charge. Anionic surfactants are negatively charged, cationic are positively charged, non-ionic possess no charge while amphoteric surfactants can be either positive or negatively charged depending on their environment and have the capacity of acting as either an acid or a base depending on the pH of the surrounding solution. Again, there are many , different species of each group and each may function in a different manner. Imidazoline-derived amphoteric surfactants are generally characterized by their relative mildness, which makes them ideal for applications in personal ,care compositions such as baby shampoo formulations. Moreover, they tend to be stable and effective over a wide pH range, and this is a useful property for many alkaline and acid cleaners used in specialty cleaner applications.

United States Patent No. 3,187,003 to McBride discloses a process for the preparation of zwitterions of 1-(2-amino-ethylimidazolines) that are useful as oil stabilizers, grease additives, fabric anti-static agents and the like. An imidazoline having an aminoethyl substituent is reacted with an α-β-unsaturated acid of from 12 to 22 carbon atoms.

United States Patent No. 2,820,043 to Rafney et al. discloses a process for the preparation of imidazoline propionic acid derivatives which are amphoteric surfactants by nature and are useful as wetting agents, penetrating agents, emulsifying agents, dispersing and cleansing agents. They are allegedly useful over a wide range of pH and are prepared by reacting a 2-hydrocarbon substituted imidazoline with a lower alkyl acrylate in the presence of heat, thus forming the lower alkyl ester of 2-substituted imidazoline propionic acid which is then hydrolyzed.

U.S. Patent No. 3,555,041 to Katz discloses a class of amphoteric imidazoline surfactants having effective surfactant properties over a wide range of pH values. These surfactants are produced by reacting long chain imidazoline compounds containing amine, alkyl, or hydroxyalkyl-substituted groups with acrylonitrile, methyl acrylate or beta propiolactone. Preferably, methyl acrylate is used.

EP 0 224 796 discloses detergent compositions comprising a phosphate surfactant and an amido-amine surfactant.

EP 0 640 334 discloses a two-part aqueous hair colouring composition comprising amphoteric surfactants including amphopropionates.

EP 0 595 383 discloses detergent compositions containing short chain amphocarboxylate detergent surfactants.

JP 10-008086 relates to lubricant compositions comprising an amido-amine acid.

DE 3 639 752 relates to processes for the preparation of amphoteric surfactants, but more particularly to the preparation of imidazoline compounds suitable as amphoteric surfactants.

EP 0 269 939 discloses detergent compositions comprising as a main detergent active ingredient a secondary amido-amine acid or its salts.

Finally, U.K. Patent No. 1,078,101 to Arndt teaches a class of amphoteric imidazolines known as 2-R-imidazoline-1-ethylene-2-oxy-propanoic acids prepared by the condensation reaction of aminoethyl ethanolamine and a fatty acid to yield an imidazoline intermediate which is then reacted with acrylic acid to yield the final product. The compounds are asserted to be useful as emulsifiers, detergents, wetting and surface active agents over a wide range of pH.

Imidazoline-based amphoteric surfactants can be divided into two groups: salt-containing and salt-free. Salt-containing imidazoline amphoteric surfactants having the general structure as shown [immediately below] are usually made from the condensation reaction of imidazoline and sodium monochloroacetate, while sodium chloride is produced as a by-product.

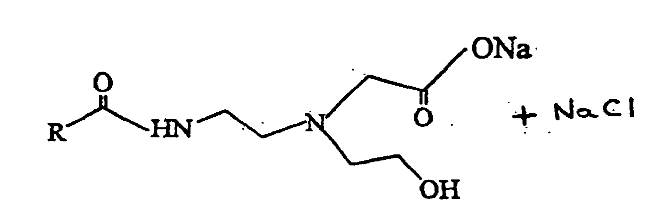

### Salt-Containing Amphoacetate

Salt-free amphoterics such as mono-amphopropionate as shown in Figure 2 have several advantages over the salt-containing counterparts in industrial applications. Salt-free amphoterics can be made by the Michael addition reaction between imidazoline with either methyl acrylate or acrylic acid under anhydrous conditions, followed by alkaline hydrolysis. Unfortunately, the reactions usually give complex mixtures as suggested by NMR, capillary electrophoresis and HPLC. Alternatively, the reactions can be carried out in aqueous media but the conversion is low. It would therefore be highly desirable to produce a salt-free amphoteric from imidazoline and acrylic acid with high amounts of mono-amphopropionate as shown below.

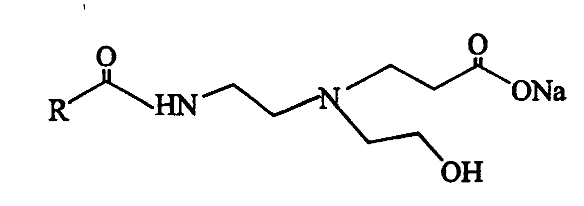

### Salt Free Amphopropionate Wherein R = C₁₁ - C₁₇ alkane

The use of acrylic acid as a reactant compound as opposed to methyl acrylate provides a number of benefits. Acrylic acid for example, has a higher flash point and is therefore safer and easier to work with. The compound also has a far less objectionable odor.

Of perhaps greater value in the process of the present invention, is that the production of salt-free amphoteric surfactants such as monoamphopropionate does not generate methanol as a by-product. Methanol is listed as a hazardous chemical by the Environmental Protection Agency (EPA). Most amphopropionate surfactants produced using methyl acrylate contain from 2.0% to over 5.0% methanol as a by-product. Storage of methyl acrylate requires expensive tanks as well as effective ventilation and absorbing equipment for removal of the vapor.

Another major problem with the production of salt-free amphopropionates using the processes known in the art is the relatively low yields of monoamphopropionates achievable. Reacting an imidazoline coco-condensate with methyl acrylate produces salt-free monoamphopropionate in yields of just 20% - 25%. This is also a very impure product with up to seven (7) different compounds produced in the reaction mixture.

### SUMMARY OF THE INVENTION

An improved process for the production of salt-free amphoteric surfactants in high yields comprises the condensation reaction of imadazoline with a mixture of acrylic acid and sodium acrylate in a molar ratio of about 1:3, respectively. The reaction is carried out in aqueous medium at elevated temperatures of from about 85°C to 100°C.

### DETAILED DESCRIPTION OF THE INVENTION

It is well known that imidazoline readily undergoes Michael addition reactions with methyl acrylate or acrylic acid under anhydrous conditions. Carbon-13 NMR analyses suggest that the reaction product after hydrolysis with sodium hydroxide contains many components rather than a single compound, the desired amphopropionate as shown in Figure 2. One possible explanation as to why Michael addition reactions afford complex mixtures is outlined in Scheme 1. In the first step, Michael addition may take place on the sp² nitrogen to give intermediate 2a, which is stabilized by the formation of 2b through the resonance mechanism.

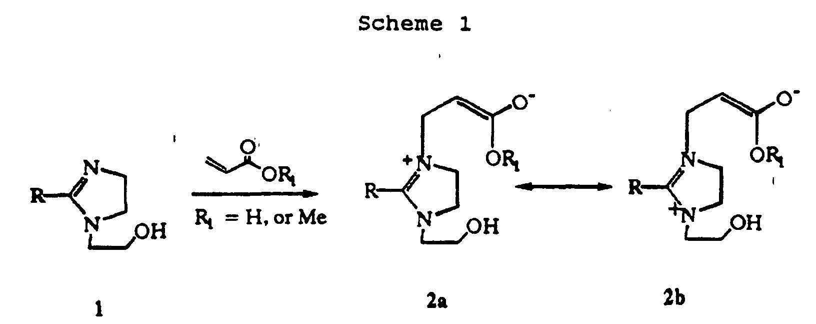

Hydrolysis of the adduct (2a) and (2b) by sodium hydroxide gives two monopropionates (3) and (4) upon cleavage of either one of the two C-N bonds. In the presence of excess alkylating reagents, (3) and (4) can be further converted to dipropionates (5) and (6), respectively. It was found that in the case of methyl acrylate, besides the nitrogen atoms in the imidazoline ring, the hydroxyl group underwent a Michael addition reaction as well. This is evidenced by the appearance of carbon-13 signals in the regions of 67 ppm.

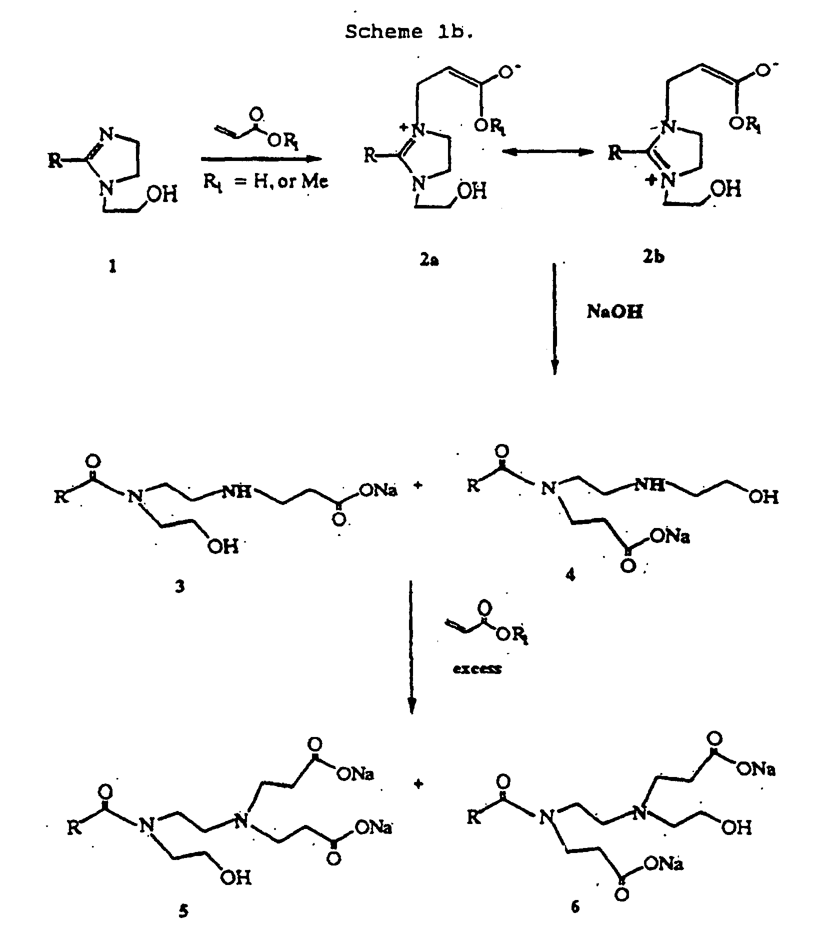

It is possible for the alkylation to occur on the sp³ nitrogen atom as well. However, the resulting intermediate (7) as shown in Scheme 2 is less stable than intermediate (2) which is stabilized by resonance structures. Consequently, the desired amphopropionate surfactant is just a minor component in the mixture.

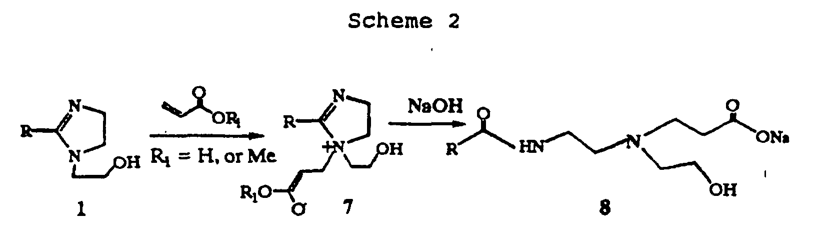

Product obtained by running the reaction in aqueous media is expected to contain more of (8) since imidazoline is known to undergo hydrolysis by water to give amidoamine (9) together with small amount of 10, which will then react with an alkylating reagent on the amine nitrogen to give (8) and (11) respectively (see Scheme 3.) The conversion of imidazoline to the product is low and the finished product contains significant amount of unreacted amidoamine (9).

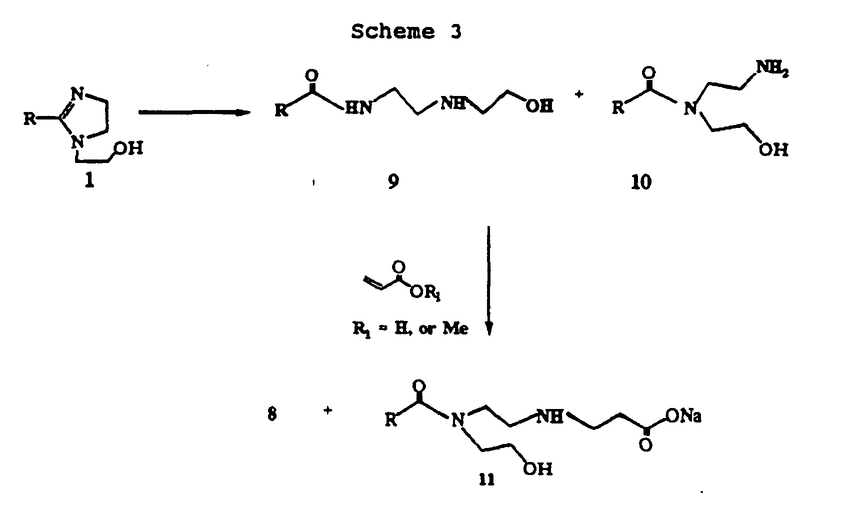

The present invention is a process to produce a salt-free amphoteric surfactant with a high content of mono-amphopropionate (8) from the readily available acrylic acid and coco-imidazoline. Clearly, a Michael addition reaction has to be utilized to produce amphopropionate (8) from acrylic acid and coco-imidazoline. However, treatment of imidazoline directly with acrylic acid would give a salt through a typical acid-base type reaction which can compete with the Michael addition reaction. One way to overcome this problem is through the use of sodium acrylate.

The Michael addition with amidoamine (9) would first give intermediate (12) as shown in Scheme 4, which then undergoes rearrangement to give (8) in relatively high yields.

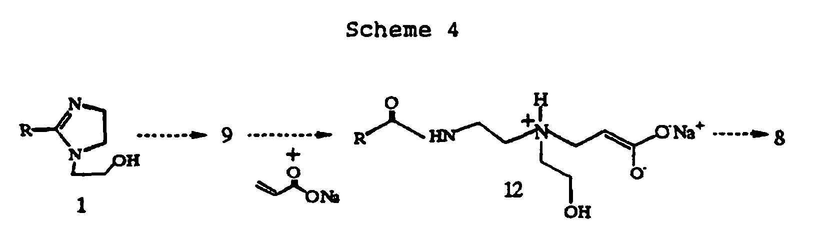

The present invention then involves the preparation of a salt-free amphoproprionate surfactant in high yields of monoamphoproprionates with few impurities and other undesirable byproducts. The process generally comprises reacting an imidazoline with a mixture of acrylic acid and sodium acrylate in an aqueous medium at elevated temperatures. The use of acrylic acid in place of methyl acrylate enables the reaction to be run without the production of methanol, an otherwise hazardous by-product. In the past, methanol was produced in amounts of up to 2.0% to 5.0% by weight of the total end product mixture.

By removing methanol as a by-product altogether, salt-free amphoteric surfactants can be produced which can be incorporated into personal care items and, in particular, cosmetic compositions where they afford superior cleaning efficacy with little to no irritation. These surfactants can also be formulated in hypoallergenic compositions which are growing in demand worldwide.

The Michael reaction occurs in an aqueous medium at elevated temperatures. The imidazoline and acrylic acid/sodium acrylate mixture are combined in a molar ratio of 1:1, i.e, equal parts imidazoline and acid/acrylate mixture. The mixture itself is comprised of acrylic acid and sodium acrylate in molar weight ratios of from about 1:6 to about 1:3. Preferably the two compounds are mixed in an amount of 25 parts acrylic acid to 75 parts sodium acrylate. The compounds are mixed together in water prior to the addition of the imidazoline. Imidazoline derivatives useful in the practice of the present invention are prepared from 2-(2-aminoethylamino)ethanol and fatty acids. Examples of fatty acids can include coconut oil fatty acids, caprylic, capric, lauric, myristic, polmitic and stearic acids.

When lauric imidazoline (Structure 1; R= C₁₁H₂₃) was treated with sodium acrylate prepared from acrylic acid and sodium hydroxide in aqueous media at 70°C, the desired Michael addition reaction did not occur after 5 hr. Carbon-13 NMR showed that imidazoline was hydrolyzed to amidoamine after 1 hr. under the reaction conditions employed. The reaction mixture was then heated to 90°C and the temperature was maintained for 20 hrs. A Carbon-13 NMR spectrum of the reaction product showed the desired Michael addition reaction had occurred and the amphopropionate surfactant (Structure 8) was formed in 37% yield based on imidazoline. The structure assignment for 8.was based on the comparison of its ¹³C-NMR spectrum with that of the well-known amphoacetate as shown in Figure 1.

The following examples are designed to better disclose the invention with more particularity in an effort to more specifically enable one to practice the process of the present invention. They are for illustrative purposes only however, and it is recognized that minor changes and alterations may be made thereto that are not contemplated herein. It is to be understood that to the extent that any such changes or alteration do not materially affect the final reaction product or results, they are to be considered as falling within the spirit and scope of the invention as defined by the claims that follow.

### Example I

To a four-neck round bottom flask equipped with a stirrer, thermometer and dropping funnel was added 268 g (1.0 mol) of coco-imidazoline, 400 g of water and a mixture of acrylic acid and sodium acrylic that was prepared in a separate vessel by adding 72 g (1.0 mol) of acrylic acid to 24 g of 50% NaOH (0.3 mol) in 200 g of water with stirring and cooling. The reaction mixture was heated to 90°C and continued for 20 hr.

The product analyzed was 38.0% solid. Analysis by carbon-13 NMR indicated the reaction produced mono-amphopropionate (8) in a 40% yield based on the amount of coco-imidazoline and 20% of unreacted amidoamine (9).

### Example II

This example illustrates that the yield of mono-amphopropionate (8) can be improved by varying the ratio of acrylic acid to sodium acrylate.

The process of Example 1 was repeated using a mixture of acrylic acid and sodium acrylate prepared from 72 g (1.0 mole) of acrylic acid and 60 g of 50% sodium hydroxide (0.75 mol) in 200 g of water. The yield of mono-amphopropionate (8) was improved to 52% based on the amount of coco-imidazoline.

### Example III

This example demonstrates that using solely sodium acrylate above does not increase the yield of mono-amphopropionate (8).

The process of Example 1 was followed using sodium acrylate prepared from 72 g (1.0 mol) of acrylate acid and 80 g of 50% sodium hydroxide (1.0 mol) in 200 g of water. The yield of amphopropionate (8) was 37% based on the amount of coco-imidazoline.

### Example IV

This example describes the procedure wherein the imidazoline is first converted to the amidoamine by sodium hydroxide and then alkylated by a mixture of acrylic acid and sodium acrylate. It also shows that the yield of amphopropionate (8) can be further increased by using an excess amount of a mixture of acrylic acid and sodium acrylate.

To a four-neck round bottom flask equipped with a stirrer, thermometer and dropping funnel was added 268 g (1.0 mol) of coco-imidazoline, 4 g of 50% NaOH (0.05 mol) aqueous solution and 200 g of water. The resulting mixture was heated at 85°C for 1 hr. with stirring. In a separate container, a mixture of acrylic acid and sodium acrylic was prepared by adding 90 g (1.25 mol) of acrylic acid to 71 g of 50% NaOH (0.89 mol) in 200 g of water with stirring and cooling. Two hundred grams of water was added to the reaction flask, followed by the mixture of acrylic acid and sodium acrylate. Heating was continued for another 16 hr and the reaction temperature was maintained at 85°C.

The product analyzed was 38.4% solids. Analysis by carbon-13 NMR indicated that an 80% yield of mono-amphopropionate (8), based on the amount of imidazoline was obtained together with less than 10% of unreacted amidoamine (9) and about 10% of unidentified components, probably dipropionates such as (5) and (6). The high content of mono-amphopropionate (8) in the product compared to a commercial product such as Miranol C2M SF from Rhone-Poulenc, Inc., was also confirmed by capillary electrophoresis. Under these conditions, the reaction conversion with respect to amidoamine was shown to be 90%. About 25% of the acrylate mixture was left unconsumed at the end of reaction as determined by NMR as well as liquid chromatography. Although it is possible to get a higher reaction conversion by using a larger access of acrylate mixture, it is certainly not desirable to have a large access of unreacted acrylate in the finished product. The finished product may contain up to 10% of dipropionates such as structures (5) and (6).

The unconsumed acrylate can be easily removed, as desired, by the treatment with an stoichiometric amount of sodium bisulfite at 85°C for 1 hour. The possible acrylic acid reformation, via a reversed Michael addition reaction, does not occur at a noticeable rate over a three-month period. This is supported by the fact that the finished product contain less than 100 ppm acrylic acid after being treated with sodium bisulfite was found to contain still less than 100 ppm of acrylic acid after 3 months at room temperature. Preferably, the reaction is carried out in the presence of air, otherwise the finished product can become cloudy which is attributed to the polymerization of acrylic acid or sodium acrylate.

### Example V

The functional surfactant characteristics of the salt-free amphoterics of the present invention were compared to those of a commercially available amphoteric, Miranol C2M SF® (Rhone-Poulenc Inc., Monmouth Jct, N.J.) a sodium cocoamphopropionate. The surface active properties of the salt-free amphoacetate were compared both before and after the amphoacetate was treated with sodium bisulfite. The results are summarized in Table 1.

**Table 1:**

| **Surface Properties of Miranol SF and Salt-Free Amphopropionate** | | | | | |
|---|---|---|---|---|---|
| Surfactant | CMC (mole/l) | γ_{cmc} (dynes/cm) | pC-20 | Foams Height (mm) (0→ 5 min) | Wetting Time (sec) |
| Miranol SF | 1.0 x 10⁻⁴ | 31.5 | 5.1 | 142 → 132 | 60 |
| Amphopropionate before Na₂SO₃ | 4.0 x 10⁻⁵ | 28.9 | 5.6 | 153 → 138 | 38 |
| Amphopropionate after Na₂SO₃ | 1.0 x 10⁻⁵ | 27.5 | 5.5 | 148 → 138 | 47 |

As shown in Table 2, compared to Miranol C2M SF, the salt-free amphopropionate either treated or untreated with sodium bisulfite is more efficient in reducing the surface tension and forming micelles. The new amphoteric surfactant also exhibits better foaming and wetting properties than Miranol C2M SF.

### Example VI

This example demonstrates an alternative procedure to that set forth in Example 1. Imidazoline was added to sodium acrylate so that a separate vessel for the preparation of acrylic acid/sodium acrylate mixture can be avoided.

To a four neck round bottom flask containing 75 g of 50% NaOH (0.94 mol) and 300 g of water was added 63.9 g (0.89 mol) of acrylic acid, followed by 268 g of coco-imidazoline. The resulting mixture was heated at 65°C for 1 hr with stirring, and then 26.1 g (0.36 mol) of acrylic acid was added. The reaction temperature was allowed to increase to 90°C and maintained at this temperature for 20 hr.

## Claims

1. A process for the preparation of an improved salt-free amphoproprionate surfactant comprising the structure 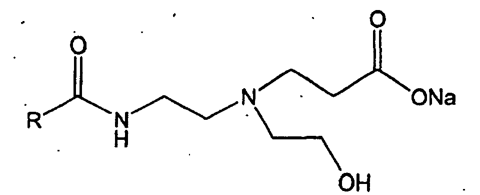 wherein R = C₇ tb C₁₇ alkane,
comprising the reaction of an imidazoline compound comprising the structure 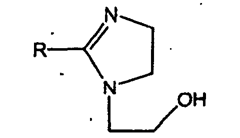 with a mixture of acrylic acid and sodium acrylate in an aqueous medium at elevated temperatures.

2. The process of claim 1 wherein said imidazoline compound is selected from the group consisting of lauric imidazoline, caprylic imidazoline, capric imidazoline, myristic imidazoline, palmitic imidazoline, stearic imidazoline, their derivatives and mixtures thereof.

3. The process of claim 2 wherein said acrylic acid and sodium acrylate are incorporated in said mixture in a range of molar ratios of from 1:6 to 1:3, respectively.

4. The process of claim 3 wherein said imidazoline compound is reacted with said acrylic acid/sodium acrylate mixture in a molar ratio of from 1:1 to 1:1.25.

5. The process of claim 4 wherein said reaction is run at a temperature of from 80°C to 100°C.

6. The process of claim 5 wherein said reaction is run at a temperature of from 85°C to 95°C.

7. The process of claim 6 further comprising the subsequent addition of sodium bisulfite in an amount sufficient to remove any excess acrylic acid.

8. The process of claim 7 wherein said reaction is carried out without the production of methanol.

## Patentansprüche

1. Verfahren zur Herstellung eines verbesserten salzfreien Amphoproprionattensids mit der folgenden Struktur: 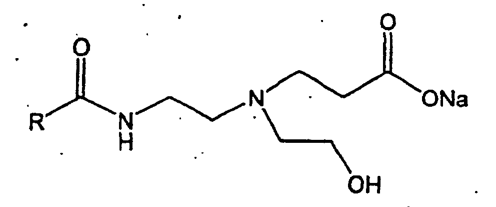 wobei R = C₇ bis C₁₇ Alkan ist,
umfassend das Reagieren einer Imidazolinverbindung mit der folgenden Struktur: 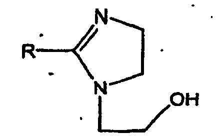 mit einem Gemisch aus Acrylsäure und Natriumacrylat in einem wässrigen Medium bei erhöhten Temperaturen.

2. Verfahren nach Anspruch 1, wobei die genannte Imidazolinverbindung ausgewählt ist aus der Gruppe bestehend aus Laurinimidazolin, Caprylimidazolin, Caprinimidazolin, Myristinimidazolin, Palmitinimidazolin, Stearinimidazolin, ihren Derivaten und Gemischen davon.

3. Verfahren nach Anspruch 2, wobei die genannte Acrylsäure und das genannte Natriumacrylat jeweils in dem genannten Gemisch in einem Molverhältnis zwischen 1:6 und 1:3 aufgenommen werden.

4. Verfahren nach Anspruch 3, wobei die genannte Imidazolinverbindung mit dem genannten Acrylsäure/Natriumacrylat-Gemisch in einem Molverhältnis zwischen 1:1 und 1:1,25 reagiert wird.

5. Verfahren nach Anspruch 4, wobei die genannte Reaktion bei einer Temperatur zwischen 80°C und 100°C abläuft.

6. Verfahren nach Anspruch 5, wobei die genannte Reaktion bei einer Temperatur zwischen 85°C und 95°C abläuft.

7. Verfahren nach Anspruch 6, ferner umfassend die nachfolgende Zugabe von Natriumbisulfit in einer Menge, die ausreicht, um überschüssige Acrylsäure zu entfernen.

8. Verfahren nach Anspruch 7, wobei die genannte Reaktion ohne Methanolproduktion durchgeführt wird.

## Revendications

1. Procédé de préparation d'un agent tensioactif d'amphoproprionate dépourvu de sel et amélioré, comprenant la structure 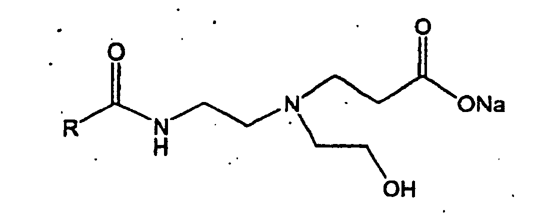 dans laquelle R = alcane en C₇ à C₁₇,
comprenant la réaction d'un composé d'imidazoline comprenant la structure 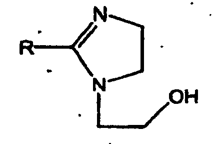 avec un mélange d'acide acrylique et d'acrylate de sodium dans un milieu aqueux à des températures élevées.

2. Procédé selon la revendication 1, dans lequel ledit composé d'imidazoline est choisi parmi le groupe constitué par l'imidazoline laurique, l'imidazoline caprylique, l'imidazoline caprique, l'imidazoline myristique, l'imidazoline palmitique, l'imidazoline stéarique, leurs dérivés et des mélanges de celles-ci.

3. Procédé selon la revendication 2, dans lequel lesdits acide acrylique et acrylate de sodium sont incorporés dans ledit mélange dans un domaine de rapports molaires allant de 1:6 à 1:3, respectivement.

4. Procédé selon la revendication 3, dans lequel ledit composé d'imidazoline est réagi avec ledit mélange acide acrylique/acrylate de sodium dans un rapport molaire allant de 1:1 à 1:1,25.

5. Procédé selon la revendication 4, dans lequel ladite réaction est réalisée à une température allant de 80°C à 100°C.

6. Procédé selon la revendication 5, dans lequel ladite réaction est réalisée à une température allant de 85°C à 95°C.

7. Procédé selon la revendication 6, comprenant en outre l'addition ultérieure de bisulfite de sodium en une quantité suffisante pour éliminer tout acide acrylique en excès.

8. Procédé selon la revendication 7, dans lequel ladite réaction est réalisée sans la production de méthanol.
